# EUROPEAN PATENT APPLICATION

(11) **EP 2 322 904 A1**
(43) Date of publication of application: **18.05.2011**
(21) Application number: 10186439.5
(22) Date of filing: 04.10.2010
(51) Int. Cl.: G01C 21/36

(54) **Navigation based on cognitive and/or physiological data**

(30) Priority: 17.11.2009 US 619866
(71) Applicant: Honeywell International Inc., Morristown, NJ 07962 (US)
(72) Inventor: Krishnaswamy, Kailash, Morristown, NJ 07962-2245 (US)
(74) Representative: Buckley, Guy Julian

(57) **Abstract**

A method of navigation comprising receiving, from at least one sensor, physiological sensor data indicative of at least one physiological attribute of a person and generating navigation-related information derived from at least some of the physiological sensor data.

## Description

### BACKGROUND

Cognitive systems are seeing increasing use in military applications. In one example, a cognitive model is used to determine the cognitive state of a solider in order to adapt the communication and/or display of information to the solider. In another example, a cognitive model is used to make friend or foe determinations.

In some such cognitive applications, navigation-related information is used as an input to a cognitive model in order to improve the quality of the outputs of the cognitive model. However, cognitive data is typically not used an input to a navigation system in order to improve the quality of the outputs of the navigation system.

### SUMMARY

One exemplary embodiment is directed to a method of navigation comprising receiving, from at least one sensor, physiological sensor data indicative of at least one physiological attribute of a person and generating navigation-related information derived from at least some of the physiological sensor data.

### DRAWINGS

FIG. 1 is a block diagram of one exemplary embodiment of a navigation system.
FIG. 2 is a flow diagram of one exemplary embodiment of a method of generating navigation information derived from physiological sensor data.

### DETAILED DESCRIPTION

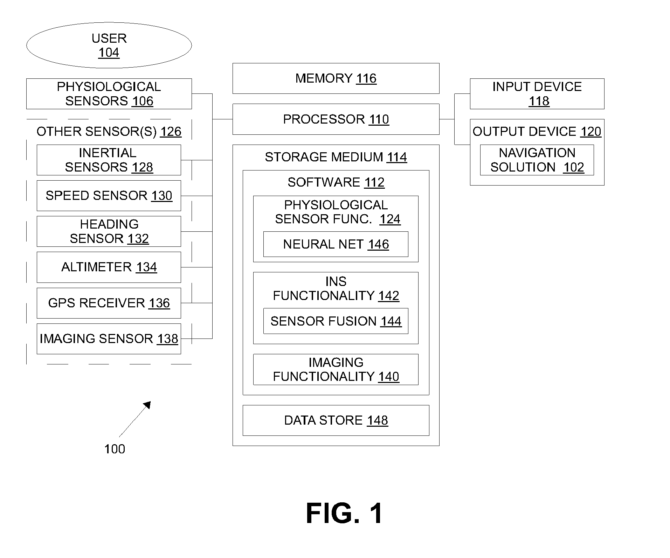

FIG. 1 is a block diagram of one exemplary embodiment of a navigation system 100. System 100 is used to generate a navigation solution 102 at least in part from physiological data associated with a person (also referred to here as the "user") 104. More specifically, in the particular exemplary embodiment shown in FIG. 1, the system 100 generates a navigation solution 102 derived from at least one attribute associated with a cognitive state or cognition of the user 104.

In the particular exemplary embodiment shown in FIG. 1, the navigation system 100 is deployed in a ground vehicle (for example, a car or truck) that is driven by the user 104. In this exemplary embodiment, the navigation system 100 is used to generate a navigation solution 102 for the vehicle as it moves through an environment based at least in part on physiological data associated with the driver of the vehicle. It is to be understood, however, that the techniques can be used in other navigation-related applications.

As used herein, a "navigation solution" comprises information about the location (position) and/or movement of the user 104 and/or vehicle. Examples of such information include information about a past, current, or future absolute location of the user 104 and/or vehicle, a past, current, or future relative location of the user 104 and/or vehicle, a past, current, or future velocity of the user 104 and/or vehicle, and/or a past, current, or future acceleration of the user 104 and/or vehicle. A navigation solution can also include information about the location and/or movement of other persons or objects within the environment of interest.

The navigation solution 102 can be used to determine the current location of the vehicle and/or user 104. The navigation solution 102 can also be used in a mapping process in which a particular environment of interest is mapped. The navigation solution 102 can also be used for other applications.

The system 100 comprises one or more physiological sensors 106 located on or near the user 104 (for example, mounted directly to the user 104, mounted to a helmet, strap, or item of clothing worn by the user 104, and/or mounted to a structure near the user 104 while the user 104 is within or near the system 100). In general, the physiological sensors 106 generate data associated with one or more physiological attributes of the user 104. More specifically, in the particular exemplary embodiment shown in FIG. 1, the physiological sensors 106 include at least one sensor that is able to measure or otherwise sense a condition or attribute that is associated with a cognitive state or cognition of the user 104. Sensor data related to such a cognitive condition or attribute is also referred to here as "cognitive sensor data". Examples of physiological sensors 106 include, without limitation, an electroencephalogram (EEG), electrocardiogram (ECG), electrooculogram (EOG), impedance pneumogram (ZPG), galvanic skin response (GSR) sensor, blood volume pulse (BVP) sensor, respiration sensor, electromyogram (EMG), blood pressure sensor, brain and body temperature sensors, neuro-infrared optical brain imaging sensor, and the like. Other physiological sensors 106 can also be used (for example, physiological sensors 106 that measure or otherwise sense a condition or attribute that is not associated with a cognitive state or cognition.

The system 100 further comprises one or more programmable processors 110 for executing software 112. The software 112 comprises program instructions that are stored (or otherwise embodied) on an appropriate storage medium or media 114 (such as flash or other non-volatile memory, magnetic disc drives, and/or optical disc drives). At least a portion of the program instructions are read from the storage medium 114 by the programmable processor 110 for execution thereby. The storage medium 114 on or in which the program instructions are embodied is also referred to here as a "program product". Although the storage media 114 is shown in FIG. 1 as being included in, and local to, the system 100, it is to be understood that remote storage media (for example, storage media that is accessible over a network or communication link) and/or removable media can also be used. The system 100 also includes memory 116 for storing the program instructions (and any related data) during execution by the programmable processor 110. Memory 116 comprises, in one implementation, any suitable form of random access memory (RAM) now known or later developed, such as dynamic random access memory (DRAM). In other embodiments, other types of memory are used.

One or more input devices 118 are communicatively coupled to the programmable processor 110 by which the user 104 is able to provide input to the programmable processor 110 (and the software 112 executed thereby). Examples of input devices include a keyboard, keypad, touch-pad, pointing device, button, switch, and microphone. One or more output devices 120 are also communicatively coupled to the programmable processor 110 on or by which the programmable processor 110 (and the software 112 executed thereby) is able to output information or data to or for the user 104. Examples of output devices 120 include visual output devices such as liquid crystal displays (LCDs), light emitting diodes (LEDs), or audio output devices such as speakers. In the exemplary embodiment shown in FIG. 1, at least a portion of the navigation solution 102 is output on the output device 120.

In the particular exemplary embodiment described here in connection with FIG. 1, the software 112 comprises physiological sensor functionality 124 that is used to determine or generate a navigation state from at least some of the physiological data output by the physiological sensors 106. As used herein, a "navigation state" refers to a particular value (or set of values) of navigation-related information. Examples of navigation states include, without limitation, a navigation state of the user 104 (such as the location, direction, speed, and/or acceleration of the user 104, the incline at which the user 104 is moving, and the stability of the user's current pose), a navigation state of a vehicle associated with the person (such as the location, direction, speed, and/or acceleration of a vehicle in which the user 104 is sitting, and/or the incline at which such a vehicle is moving), a navigation state of an object or person associated with the user 104 (such as the location, direction, speed, and/or acceleration of an object or person near the user 104), the identity and/or location of landmarks within an environment of interest, and whether an entity (such as a vehicle or person) is a friend or foe.

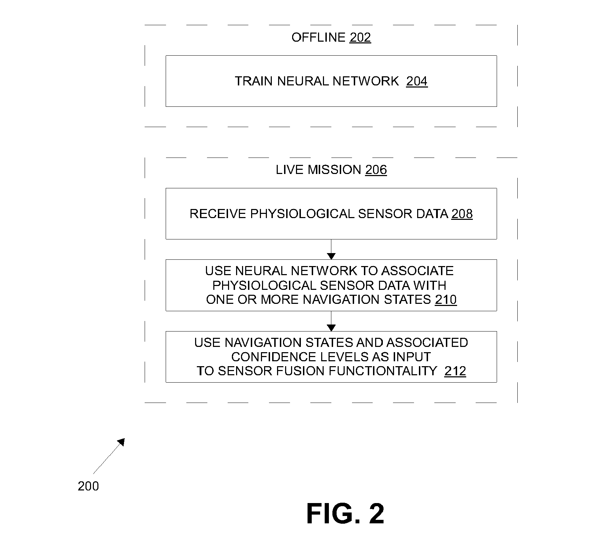

One exemplary embodiment of a method of determining or generating a navigation state from at least some of the physiological data output by the physiological sensors 106 is described below in connection with FIG. 2. That exemplary method uses a neural network 146 implemented as a part of the physiological sensor functionality 124 to associate one or more navigation states with a set of physiological sensor data output by the physiological sensors 106.

In the particular exemplary embodiment shown in FIG. 1, the system 100 includes, in addition to the physiological sensors 106, one or more other sensors 126. The other sensors 126, in this exemplary embodiment, include one or more inertial sensors 128 (such as accelerometers and gyroscopes), one or more speed sensors 130, one or more heading sensors 132, one or more altimeters 134, one or more GPS receivers 136, and one or more imaging sensors 138 (such as three-dimensional (3D) light detection and ranging (LIDAR) sensors, stereo vision cameras, millimeter wave RADAR sensors, and ultrasonic range finders). In other embodiments, however, other combinations of sensors can be used.

In the exemplary embodiment shown in FIG. 1, the software 112 also comprises inertial navigation system (INS) functionality 142 that generates the navigation solution 102. The INS functionality 142 generates navigation information (also referred to here as "inertial navigation information") from the sensor data output by the inertial sensors 128 (such as accelerometers and gyroscopes), speed sensor 130, heading sensors 13, and altimeter 134 in a conventional manner using techniques known to one of ordinary skill in the art.

In this exemplary embodiment, the software 112 also comprises imaging functionality 140 that generates navigation information (also referred to here as "imaging navigation information") from the imaging sensor data generated by the imaging sensors 138. The imaging navigation information is generated in a conventional manner using techniques known to one of ordinary skill in the art.

In this exemplary embodiment, the INS functionality 142 also comprises sensor fusion functionality 144. As a part of generating the navigation solution 102, the sensor fusion functionality 144 combines the navigation information generated by the physiological sensor functionality 124, the inertial navigation information generated by the INS functionality 142, the imaging navigation information generated by the imaging functionality 140, and GPS data generated by the GPS receiver 136. In this exemplary embodiment, the navigation information generated by the physiological sensor functionality 124, the inertial navigation information generated by the INS functionality 142, the imaging navigation information generated by the imaging functionality 140, and the GPS data generated by the GPS receiver 136 each have a respective associated confidence level or uncertainty estimate that the sensor fusion functionality 144 uses to combine such navigation-related information. In one implementation of such an exemplary embodiment, the sensor fusion functionality 144 comprises a Kalman filter. In other implementations, other sensor fusion techniques are used.

By using the cognitive and other physiological information derived from physiological data generated by the physiological sensors 106 as an input, the sensor fusion functionality 144 can use this cognitive and other physiological information to improve the navigation solution 102 output by the INS functionality 142. For example, in environments where GPS is not available and/or where the imaging sensors are not reliable or operable, the navigation information derived from the physiological sensors 106 can still be used to control inertial navigation information error growth.

In the particular exemplary embodiment shown in FIG. 1, the system 100 further comprises a data store 148 to and from which various types of information can be stored and read in connection with the processing the software 112 performs.

FIG. 2 is a flow diagram of one exemplary embodiment of a method 200 of generating navigation information derived from physiological sensor data. The exemplary embodiment of method 200 shown in FIG. 2 is described here as being implemented using the system 100 of FIG. 1, though other embodiments can be implemented in other ways. In particular, the exemplary embodiment of method 200 shown in FIG. 2 is implemented by the physiological sensor functionality 124 and the INS functionality 142. Also, in the particular embodiment shown in FIG. 2, at least a portion of the physiological sensor data is indicative of a cognitive state or cognition of the user 104.

In the particular exemplary embodiment described here in connection with FIG. 2, the physiological sensor functionality 124 uses a neural network 146. The neural network 146 is configured in a conventional manner using techniques known to one of ordinary skill in the art.

Method 200 comprises, as a part of an offline process 202 performed prior to the system 100 being used on a live mission, training the neural network 146 using a set of training data (block 204). The set of training data is obtained by having the user 104 perceive various navigation-related experiences or stimuli and capturing the physiological sensor data that is generated by the physiological sensors 106 in response to each such experiences or stimuli. The training data can be captured in an off-line process performed in a controlled environment and/or during "live" missions where the user 104 has perceived the relevant navigation-related experiences or stimuli. Examples of such navigation-related experiences or stimuli include, without limitation, positioning the user 104 at various locations within an environment of interest, moving the user 104 in or at various directions, inclines, speeds, and/or rates of acceleration, having the user 104 view various objects of interest while positioned at various locations within the environment of interest, having the user 104 view various objects of interest while moving in or at various directions, inclines, speeds, and/or rates of acceleration, having the user 104 view various landmarks of interest while positioned at various locations within the environment of interest, viewing various "friendly" and "foe" vehicles or persons.

In this exemplary embodiment, individual training data is captured for particular users 104 of the system 100 so that each such user 104 has a separate instantiation of the neural network 146 that is trained with the individual training data that has been captured specifically for that user 104. Training data can also be captured for several users (for example, users of a particular type or class) and the captured data can be used to train a single instantiation of the neural network 146 that is used, for example, for users of that type or class. This latter approach can also be used to create a "default" instantiation of the neural network 146 that is used, for example, in the event that a user 104 for whom no other neural network instantiation is available uses the system 100.

The captured training data is used to train the neural network 146 in a conventional manner using techniques known to one of ordinary skill in the art.

Method 200 further comprises, during a live mission 206, receiving physiological sensor data from one or more of the physiological sensors 106 (block 208) and using the trained neural network 146 to associate one or more navigation states (or other navigation-related information) with a particular set of the received physiological sensor data (block 212). In the particular exemplary embodiment described here in connection with FIG. 2, the current values output by the physiological sensors 106 are input to the neural network 146, which in turn produces an output that comprises one or more navigation states that the neural network 146 indicates are associated with the current values output by the physiological sensors 106. The output of the neural network 146, in this exemplary embodiment, also comprises a respective confidence level for each such navigation state. For each navigation state output by the neural network 146, the confidence level indicates how closely the set of inputs (that is, the current values output by the physiological sensors 106) matches the training input data associated with that navigation state.

Method 200 further comprises, during a live mission 206, using the set of navigation states and associated confidence levels output by the neural network 146 as inputs to the sensor fusion functionality 144 (block 212). In the particular exemplary embodiment described here in connection with FIG. 2, the sensor fusion functionality 144 combines the set of navigation states and associated confidence levels output by the neural network 146 with the inertial navigation information generated by the INS functionality 142, the imaging navigation information generated by the imaging functionality 140, and GPS data generated by the GPS receiver 136 as a part of generating the navigation solution 102. In this exemplary embodiment, as noted above, the inertial navigation information generated by the INS functionality 142, the imaging navigation information generated by the imaging functionality 140, and the GPS data generated by the GPS receiver 136 also each have their own respective associated confidence levels or uncertainty estimates that the sensor fusion functionality 144 uses to combine such navigation-related information. In one implementation of such an exemplary embodiment, the sensor fusion functionality 144 comprises a Kalman filter.

As noted above, by using the cognitive and other physiological information derived from the physiological sensor data generated by the physiological sensors 106 as an input, the sensor fusion functionality 144 can use this cognitive and other physiological information to improve the navigation solution 102 output by the INS functionality 142 (for example, to control inertial navigation information error growth in environments where GPS is not available and/or where the imaging sensors are not reliable or operable).

In the particular exemplary embodiment described above in connection with FIGS. 1-2, a neural network is used to generate or determine one or more navigation states derived from at least some of the physiological sensor data. However, in other embodiments, one or more navigation states are generated from at least some of the physiological sensor data in other ways. For example, parametric techniques (such as linear regression, generalized linear regression, logistic regression and discriminant analysis), recursive partitioning techniques (such as classification tree methods), and non-parametric techniques (such as genetic algorithms and k-nearest neighbor algorithms) and/or combinations thereof can be used. Also, other techniques can be used.

The methods and techniques described here may be implemented in digital electronic circuitry, or with a programmable processor (for example, a special-purpose processor or a general-purpose processor such as a computer) firmware, software, or in combinations of them. Apparatus embodying these techniques may include appropriate input and output devices, a programmable processor, and a storage medium tangibly embodying program instructions for execution by the programmable processor. A process embodying these techniques may be performed by a programmable processor executing a program of instructions to perform desired functions by operating on input data and generating appropriate output. The techniques may advantageously be implemented in one or more programs that are executable on a programmable system including at least one programmable processor coupled to receive data and instructions from, and to transmit data and instructions to, a data storage system, at least one input device, and at least one output device. Generally, a processor will receive instructions and data from a read-only memory and/or a random access memory. Storage devices suitable for tangibly embodying computer program instructions and data include all forms of non-volatile memory, including by way of example semiconductor memory devices, such as EPROM, EEPROM, and flash memory devices; magnetic disks such as internal hard disks and removable disks; magnetooptical disks; and DVD disks. Any of the foregoing may be supplemented by, or incorporated in, specially-designed application-specific integrated circuits (ASICs).

A number of embodiments of the invention defined by the following claims have been described. Nevertheless, it will be understood that various modifications to the described embodiments may be made without departing from the spirit and scope of the claimed invention. Accordingly, other embodiments are within the scope of the following claims.

## Claims

1. A method of navigation comprising:
receiving, from at least one sensor, physiological sensor data indicative of at least one physiological attribute of a person; and
generating navigation-related information derived from at least some of the physiological sensor data.

2. The method of claim 1, wherein at least some of the physiological sensor data is indicative of at least one of a cognitive state of the person and cognition of the person.

3. The method of claim 1, wherein the navigation-related information comprises at least one of: a navigation solution; a navigation state for the person; a navigation state of a vehicle associated with the person; a navigation state of an object associated with the person; an identity of a landmark; a location of a landmark; and whether an entity is a friend or foe; and

4. The method of claim 1, wherein generating the navigation-related information as a function of at least some of the physiological data comprises associating at least some of the physiological data with a navigation state.

5. The method of claim 1 further comprising receiving other sensor data from at least one other sensor, wherein the navigation-related information is generated from at least some of the physiological sensor data and at least some of the other sensor data, and wherein at least some of the physiological sensor data is used as an input to a sensor fusion process in connection with generating the navigation-related information.

6. An apparatus comprising:
at least one physiological sensor to generate physiological sensor data indicative of at least one physiological attribute of a person;
a processor communicatively coupled to the sensor, wherein the processor is configured to generate navigation-related information derived from at least some of the physiological sensor data.

7. The apparatus of claim 6, wherein at least some of the physiological sensor data is indicative of at least one of a cognitive state of the person and cognition of the person.

8. The apparatus of claim 6, wherein the processor is configured to generate the navigation-related information derived from at least some of the physiological sensor data by associating at least some of the physiological sensor data with a navigation state.

9. The apparatus of claim 6, further comprising at least one other sensor to generate other sensor data, wherein the processor is configured to generate the navigation-related information derived from at least some of the physiological sensor data by generating a navigation solution derived from at least some of the physiological data and at least some of the other sensor data.

10. The apparatus of claim 9, wherein the other sensors comprise at least one of: an inertial sensor, a speed sensor, a heading sensor, an altimeter, a global position satellite (GPS) receiver, and an imaging sensor.
